# EUROPÄISCHE PATENTSCHRIFT

(11) **EP 3 918 200 B1**
(45) Veröffentlichungstag und Bekanntmachung des Hinweises auf die Patenterteilung: **03.04.2024**
(21) Anmeldenummer: 20702761.6
(22) Anmeldetag: 27.01.2020
(51) Int. Cl.: F04C 2/10, F04C 15/00

(54) **ZAHNRADPUMPE**
GEAR PUMP
POMPE À ENGRENAGE

(30) Priorität: 28.01.2019 DE 102019102073
(43) Veröffentlichungstag der Anmeldung: 08.12.2021
(73) Patentinhaber: Fresenius Medical Care Deutschland GmbH, 61352 Bad Homburg (DE)
(72) Erfinder: LIPPERT, Simone, 97737 Gemünden am Main (DE)
(74) Vertreter: Herrmann, Uwe
(86) Internationale Anmeldenummer: PCT/EP2020/051944
(87) Internationale Veröffentlichungsnummer: WO 2020/157015

(56) Entgegenhaltungen:
- EP-A1- 3 115 609
- JP-A- S60 178 986
- US-A- 3 986 797
- US-A1- 2005 042 124

## Beschreibung

Die vorliegende Erfindung betrifft eine Zahnradpumpe mit einem Gehäuse, das einen Zulauf für die zu fördernde Flüssigkeit und einen Ablauf für die geförderte Flüssigkeit sowie einen Pumpenraum aufweist, in dem sich das oder die Zahnräder zur Förderung der Flüssigkeit befinden, wobei sich in dem Gehäuse eine Lagerstelle befindet, in der das oder die Zahnräder bzw. deren Achsen drehbar aufgenommen ist.

Zahnradpumpen sind Verdrängerpumpen, bei denen die Flüssigkeit durch die Bewegung der Zahnräder von der Saugseite zu der Druckseite gefördert wird. Üblicherweise weisen Zahnradpumpen zwei ineinander greifende Zahnräder auf, von der Erfindung sind jedoch auch Pumpen umfasst, die nur ein Zahnrad aufweisen und bei denen die Verdrängung der Flüssigkeit zwischen dem Zahnrad und einem Gehäuseteil erfolgt, in dem das Zahnrad drehbar aufgenommen ist.

Das oder die Zahnräder bzw. deren Achsen sind bei bekannten Zahnradpumpen üblicherweise in Gleitlagern drehbar aufgenommen, wobei die Gleitlager mit Schmiermittel versehen sind, um einen reibungs- und geräuscharmen Betrieb zu gewährleisten. Dies ist allerdings mit dem Nachteil verbunden, dass Schmiermittel in die zu fördernde Flüssigkeit gelangen kann, was insbesondere bei medizinischen Anwendungen nicht gewünscht ist.

US 2005/042124 A1 bezieht sich auf eine kleine Zahnradpumpe, die für Geräte und Systeme wie ein Kühlsystem für elektronische Geräte verwendet wird. US 3 986 797 A offenbart eine magnetisch angetriebene Zahnradpumpe zur Verwendung in einer künstlichen Nierenmaschine. JP S60 178986 A offenbart eine Zahnradpumpe für medizinische Zwecke. Eine ähnliche Pumpe wurde auch von EP3115609A1 offenbart.

Der vorliegenden Erfindung liegt daher die Aufgabe zugrunde, eine Zahnradpumpe der eingangs genannten Art dahingehen weiterzubilden, dass die Gefahr der Verunreinigung der zu fördernden Flüssigkeit mit Schmiermittel verhindert wird.

Diese Aufgabe wird durch eine Zahnradpumpe mit den Merkmalen des Anspruchs 1 und des Anspruchs 2 gelöst.

Danach ist vorgesehen, dass sich der Zulauf in einer anderen Ebene befindet als der Ablauf und dass mit dem Zulauf und/oder mit dem Ablauf unmittelbar oder mittelbar ein erster Spülkanal in Fluidverbindung steht, der sich bis zu der Lagerstelle hin erstreckt. Die Lagerstelle wird somit selbstständig von der zu fördernden oder geförderten Flüssigkeit (im Folgenden als "Förderflüssigkeit" bezeichnet) umspült, die somit gleichzeitig als Schmiermittel dient. Ein Trockenlauf der Lagerstelle(n) wird somit wirksam auch ohne die Verwendung eines separaten Schmiermittels verhindert.

Der erste Spülkanal erstreckt sich von dem Zulauf oder von dem Ablauf oder von einem mit diesen in Fluidverbindung stehenden Kanälen und mündet in der oder in den Lagerstelle(n) für die Zahnrädern, wobei die Niveaudifferenz zwischen Zulauf und Ablauf die Möglichkeit für die Schaffung eines Fallkanals bietet, der immer dann durchströmt wird, wenn die Pumpe in Betrieb ist. Vorzugsweise zweigt von dem Fallkanal der erste Spülkanal ab. Ebenfalls vorzugsweise erstreckt sich der Fallkanal von der Druckseite der Pumpe zum Ablauf.

In einer weiteren Variante ist vorgesehen dass sich in oder an dem Gehäuse ein Magnet zum Antrieb der Zahnräder befindet und dass sich auf der Druckseite der Zahnräder ein zweiter Spülkanal befindet, der sich bis zu dem Magneten hin erstreckt. Dieser zweite Spülkanal wird ebenfalls durchströmt, wenn sich die Pumpe in Betrieb befindet, wobei die Förderflüssigkeit bis hin in den Drehbereich des Magneten gelangt, so dass dieser von Medium umspült wird und kein Trockenlauf im Bereich der Kopplung zu dem oder den Zahnrädern entsteht. Auch hier wird somit kein separates Schmiermittel benötigt.

Auch eine Kombination der beiden vorgenannten Gedanken ist denkbar, so dass die Zahnradpumpe nach Anspruch 1 mit den Merkmalen des Anspruchs 2 ausgeführt sein kann.

Vorzugsweise ist der Zulauf höher angeordnet als der Ablauf und es ist ein Fallkanal vorgesehen, der sich über die Niveaudifferenz oder einen Teil von dieser zwischen Zulauf und Ablauf erstreckt, wobei der Fallkanal mit dem oder den Spülkanälen in Fluidverbindung steht.

Vorzugsweise handelt es sich bei der oder den Lagerstellen für die Achsen der Zahnräder um Gleitlager.

Das Gehäuse der Zahnradpumpe kann aus mehreren einzelnen Schichten aufgebaut sein, beispielsweise aus Metallplatten oder dergleichen. Es können zwei oder drei getrennte Schichten vorgesehen sein, die miteinander verbunden werden, was eine leichte Fertigung ermöglicht sowie eine leichte Demontage bei geringen Möglichkeiten der Verunreinigung.

Denkbar ist es, dass eine Schicht den Gehäusegrundkörper und eine Schicht einen Deckel bzw. eine Deckelschicht bildet, wobei in dem Gehäusegrundkörper der Zulauf, der Pumpenraum und der Ablauf angeordnet sind und wobei in der Deckelschicht der Magnet zum Antrieb des oder der Zahnräder angeordnet ist.

Der Pumpenraum befindet sich auf Höhe des Zulaufs und der Ablauf unterhalb dieses Niveaus. Vorzugsweise stehen der erste und der zweite Spülkanal miteinander in Fluidverbindung, so dass die Förderflüssigkeit im Betrieb der Pumpe sowohl das oder die Lager für die Zahnräder als auch den Bereich zwischen Magnet und Zahnrad bzw. den Zahnrädern umspült.

Die vorliegende Erfindung betrifft des Weiteren die Verwendung einer Zahnradpumpe gemäß der Erfindung zur Förderung biologischer bzw. medizinischer Flüssigkeiten, insbesondere zur Förderung von Flüssigkeit, die zur Herstellung einer Dialyselösung verwendet wird, wie z.B. Wasser, oder zur Förderung einer gebrauchsfertigen Dialyselösung.

Die vorliegende Erfindung betrifft des Weiteren die Verwendung einer Zahnradpumpe gemäß der Erfindung in einem Blutbehandlungsgerät, insbesondere in einem Dialysegerät. Vorzugsweise dient die Zahnradpumpe als Entgasungspumpe. Des Weiteren betrifft die vorliegende Erfindung ein Blutbehandlungsgerät, insbesondere Dialysegerät mit einer Zahnradpumpe gemäß der Erfindung.

Die vorstehenden Ausführungen bzgl. der Lage der Elemente der Pumpe, wie Zufluss, Abfluss, Pumpenraum etc. beziehen sich auf die Pumpe im Gebrauchszustand. Dies ist der Zustand, in dem die Pumpe vertikal steht, wobei in diesem Zustand Zulauf und Ablauf horizontal verlaufen und sich auch die Zahnräder in einer horizontalen Ebene befinden. Vorzugsweise verläuft der erste Spülkanal seitlich, wie z.B. horizontal und der zweite Spülkanal nach oben, vorzugsweise vertikal. Vorzugsweise befindet sich der Magnet oberhalb des oder der Zahnräder.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1:: eine Schnittansicht durch die Zahnradpumpe,
- Figur 2:: eine weitere Schnittansicht der Zahnradpumpe mit ersten Spülkanälen,
- Figur 3:: eine Schnittansicht durch den Gehäusegrundkörper,
- Figur 4:: eine weitere Schnittansicht der Zahnradpumpe mit Dichtungsbereichen und
- Figur 5:: perspektivische Ansichten des Gehäuses und des Gehäusegrundkörpers.

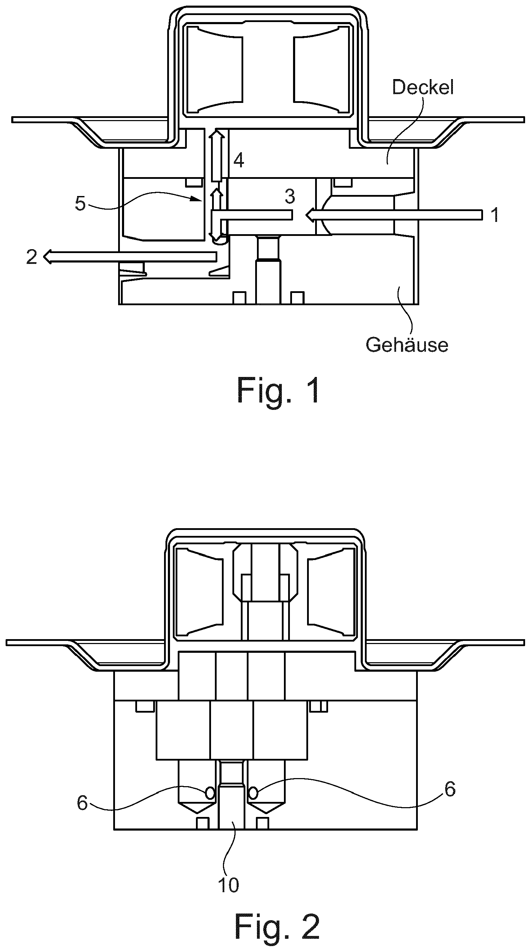

Figur 1 zeigt eine Zahnradpumpe gemäß der Erfindung mit dem Gehäusegrundkörper G und mit der Deckelschicht D, die auf den Gehäusegrundkörper G fluiddicht aufgesetzt ist.

In dem Gehäusegrundkörper G befindet sich der Einlass bzw. Zulauf 1 für das zu fördernde Medium, der zu dem Pumpenraum 3 führt, in dem sich die nicht dargestellten Zahnräder befinden. Auf der Druckseite des Pumpenraums 3, d.h. gemäß Figur 1 links von diesem befindet sich der Kanal 5, der vertikal angeordnet ist und der in den Auslass bzw. Ablauf 2 mündet.

In der Deckelschicht D befindet sich der zweite Spülkanal 4, der zu dem oberhalb der Deckelschicht D befindlichen Magneten M führt. Der Magnet wirkt mit wenigstens einem der Zahnräder so zusammen, dass eine Drehbewegung des Magneten zu einer Drehbewegung des oder der Zahnräder führt.

Durch den Einlass 1 wird Flüssigkeit durch das Gehäuse in den Pumpenraum 3 gefördert. Dieses verteilt sich durch die Drehung des oder der Zahnräder im Pumpenraum 3 und gelangt über den zweiten Spülkanal bzw. Zuführkanal 4 im Deckel D in den Drehbereich des Magneten M, so dass dieser von der Flüssigkeit umspült wird und kein Trockenlauf im Bereich der Kopplung zum Zahnrad entsteht. Die Kanäle 4 und 5 liegen genau übereinander, so dass optimale Fluss- und Strömungsverhältnisse gewährleistet sind.

Von dem Kanal gelangt somit ein Teil der Förderflüssigkeit in den Ablauf 2 und einen Teil über den zweiten Spülkanal 4 in den Bereich des Magneten M.

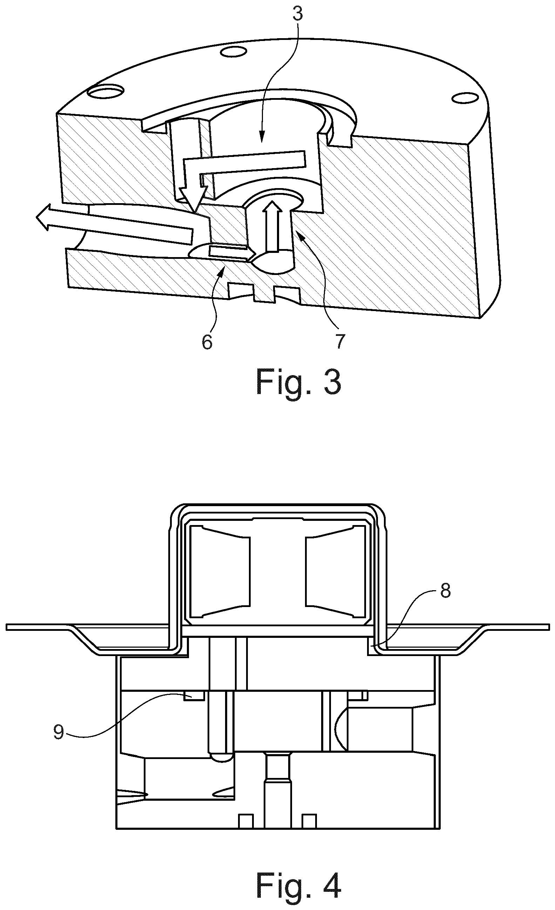

Figur 2 zeigt eine Ansicht der Pumpe, in der die ersten Spülkanäle 6 sichtbar sind, deren Anordnung besser aus Figur 3 ersichtlich wird.

Durch den Höhenversatz zwischen Zulauf 1 und Ablauf 2 gelangt die Förderflüssigkeit automatisch über den Fallkanal 5 zum Auslass, der auf einem tieferen Niveau liegt als der Einlass und als die Pumpenkammer 3, ohne dass dazu eine Bewegung des oder der Zahnräder erforderlich wäre. Der Fallkanal 5 dient als Versorgungsdurchgang für den ersten Spülkanal 6, der wie alle anderen Kanäle als Bohrung ausgeführt sein kann.

Der erste Spülkanal 6 steht in Fluidverbindung mit der Gleitlagerstelle 7 und versorgt diese mit Förderflüssigkeit von unten. Von dort gelangt die Flüssigkeit in den Pumpenraum 3 und dann wieder zurück in den Fallkanal 5, so dass eine Kreislaufströmung entsteht.

Dadurch wird gewährleistet, dass die Gleitlagerstelle 7 stets mit Flüssigkeit versorgt ist und ein separates Schmiermittel nicht erforderlich ist. Dies wird sich positiv auf die Gleitlagerstelle(n) 7 aus.

Vorzugsweise weist die Pumpe gemäß der Erfindung (außer der der Förderflüssigkeit) kein Schmiermittel auf.

Die Drehbewegung der Welle des oder der Zahnräder in der Gleitlagerbohrung 7 und der Zufluss immer neuer Flüssigkeit durch die erste Spülleitung 6 versorgen die Lagerstelle 7 mit immer frischem Medium, was gleichzeitig das Schmiermittel für die Lagerstelle 7 ist und sich durch diesen Effekt selbst erneuert.

Durch den kontinuierlichen Fluss ist die Förderflüssigkeit in allen funktionsrelevanten Bereichen der Pumpe ständig in Bewegung. Somit werden die relevanten Bereiche stetig umspült und es kommt zu keiner Keimbildung durch tote Äste oder sonstige nicht vorhandene Totzonen.

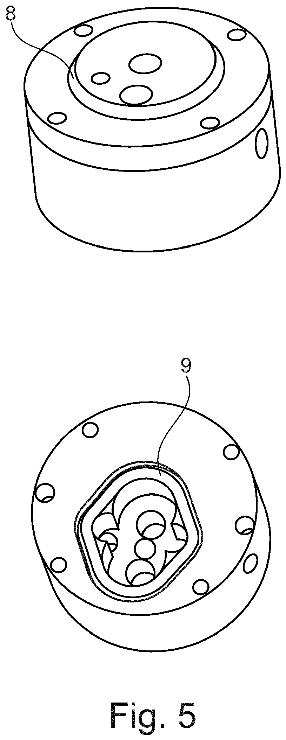

Figur 5 zeigt die Anordnung von Dichtring 8 und Dichtung 9 auf dem Gehäusegrundkörper G (rechte Darstellung) bzw. auf dem Deckel D (linke Darstellung), wobei die Dichtung 9 zwischen dem Gehäusegrundkörper G und dem Deckel D abdichtet und wobei die Dichtung 8 zwischen dem Deckel D und einer Abdeckung A der Zahnradpumpe, d.h. nach außen hin abdichtet.

Die Position der Dichtungen 8, 9 ist so konzipiert, dass die Entstehung von Totzonen ausgeschlossen ist. Ebenfalls ausgeschlossen ist eine Spaltbildung zwischen Deckel D und Gehäusegrundkörper G durch die sehr enge Positionierung der Dichtung 9, die um den Pumpenraum 3 umläuft. Die sehr kleine Fläche wird durch die Bewegung der Flüssigkeit ständig gespült, eine Keimbildung tritt somit nicht auf.

Denkbar ist es, einen Bypasskanal 10 (vgl. Figur 2) vorzusehen, der zur Druckregulierung der Pumpe dient. Dieser Bypasskanal erstreckt sich vom Zulauf durch die beiden ersten Spülleitungen 6 zum Ablauf 2. Die Druckregulierung erfolgt über eine Bypassschraube, die in die Bypassbohrung 10 eingesetzt ist. Deren Abdichtung erfolgt über eine Geometrie an der Bypassschraube, so dass der Einsatz eines O-Rings nicht erforderlich ist.

## Patentansprüche

1. Zahnradpumpe mit einem Gehäuse, das einen Zulauf (1) für die zu fördernde Flüssigkeit und einen Ablauf (2) für die geförderte Flüssigkeit sowie einen Pumpenraum (3) aufweist, in dem sich das oder die Zahnräder zur Förderung der Flüssigkeit befinden, wobei sich in dem Gehäuse wenigstens eine Lagerstelle (7) befindet, in der wenigstens ein Zahnrad drehbar aufgenommen ist, wobei sich der Zulauf (1) in einer anderen Ebene befindet als der Ablauf (2) und dass mit dem Zulauf (1) und/oder mit dem Ablauf (2) unmittelbar oder mittelbar zumindest ein erster Spülkanal (6) in Fluidverbindung steht, der sich bis zu der oder den Lagerstellen (7) hin erstreckt, **dadurch gekennzeichnet, dass** in dem Gebrauchszustand, in dem die Zahnradpumpe vertikal steht und der Zulauf (1) und der Ablauf (2) horizontal verlaufen, sich der Pumpenraum (3) auf Höhe des Zulaufs (1) und der Ablauf (2) unterhalb dieses Niveaus befindet.

2. Zahnradpumpe mit einem Gehäuse, das einen Zulauf (1) für die zu fördernde Flüssigkeit und einen Ablauf (2) für die geförderte Flüssigkeit sowie einen Pumpenraum (3) aufweist, in dem sich das oder die Zahnräder zur Förderung der Flüssigkeit befinden, wobei sich in dem Gehäuse zumindest eine Lagerstelle (7) befindet, in der wenigstens eines der Zahnräder drehbar aufgenommen ist, und wobei sich in dem Gehäuse ein Magnet (M) zum Antrieb des oder der Zahnräder befindet, wobei sich auf der Druckseite der Zahnräder ein zweiter Spülkanal (4) befindet, der sich bis zu dem Magneten (M) hin erstreckt, **dadurch gekennzeichnet, dass** in dem Gebrauchszustand, in dem die Zahnradpumpe vertikal steht und der Zulauf (1) und der Ablauf (2) horizontal verlaufen, sich der Pumpenraum (3) auf Höhe des Zulaufs (1) und der Ablauf (2) unterhalb dieses Niveaus befindet.

3. Zahnradpumpe nach Anspruch 1, **dadurch gekennzeichnet, dass** die Zahnradpumpe mit den Merkmalen des Anspruch 2 ausgeführt ist.

4. Zahnradpumpe nach einem der vorhergehenden Ansprüche, **dadurch gekennzeichnet, dass** der Zulauf (1) höher angeordnet ist als der Ablauf (2) und dass ein Fallkanal (5) vorgesehen ist, der sich über die Niveaudifferenz oder einen Teil von dieser zwischen Zulauf (1) und Ablauf (2) erstreckt, wobei der Fallkanal (5) mit dem oder den Spülkanälen (4, 6) in Fluidverbindung steht.

5. Zahnradpumpe nach einem der vorhergehenden Ansprüche, **dadurch gekennzeichnet, dass** es sich bei der Lagerstelle (7) um ein Gleitlager handelt.

6. Zahnradpumpe nach einem der vorhergehenden Ansprüche, **dadurch gekennzeichnet, dass** das Gehäuse aus mehreren einzelnen Schichten aufgebaut ist.

7. Zahnradpumpe nach dem Anspruch 2 und dem Anspruch 6, und einem der Ansprüche 3 - 5, **dadurch gekennzeichnet, dass** eine Schicht den Gehäusegrundkörper und eine Schicht eine Deckelschicht bildet, wobei in dem Gehäusegrundkörper der Zulauf (1), der Pumpenraum (3) und der Ablauf (2) angeordnet sind und wobei in der Deckelschicht der Magnet (M) zum Antrieb des oder der Zahnräder angeordnet ist.

8. Zahnradpumpe nach dem Anspruch 3 und einem der Ansprüche 4 bis 7, **dadurch gekennzeichnet, dass** der erste (6) und der zweite (4) Spülkanal miteinander in Fluidverbindung stehen.

9. Verwendung einer Zahnradpumpe gemäß einem der Ansprüche 1 bis 8 zur Förderung biologischer bzw. medizinischer Flüssigkeiten, insbesondere zur Förderung von Flüssigkeit zur Herstellung einer Dialyselösung oder zur Förderung einer Dialyselösung.

10. Verwendung einer Zahnradpumpe gemäß einem der Ansprüche 1 bis 8 in einem Blutbehandlungsgerät, insbesondere in einem Dialysegerät.

11. Verwendung nach Anspruch 9 oder 10, **dadurch gekennzeichnet, dass** die Zahnradpumpe als Entgasungspumpe dient.

12. Blutbehandlungsgerät, insbesondere Dialysegerät mit einer Zahnradpumpe nach einem der Ansprüche 1 bis 8.

## Claims

1. Gear pump having a housing which comprises an inflow (1) for the liquid to be conveyed, an outflow (2) for the conveyed liquid, and a pump chamber (3) in which the gear or gears for conveying the liquid are present, wherein at least one bearing position (7) is present in the housing, in which bearing position at least one gear is rotatably received, wherein the inflow (1) is in a different plane than the outflow (2); and in that at least one first flushing passage (6) that extends up to the bearing position(s) (7) is in direct or indirect fluid communication with the inflow (1) and/or with the outflow (2), **characterized in that** in the use state, in which the gear pump is in vertical position and the inflow (1) and the outflow (2) extend horizontally, the pump chamber (3) is located at the level of the inflow (1) and the outflow (2) is located below this level.

2. Gear pump having a housing which comprises an inflow (1) for the liquid to be conveyed, an outflow (2) for the conveyed liquid, and a pump chamber (3) in which the gear or gears for conveying the liquid are present, wherein at least one bearing position (7) is present in the housing, in which bearing position at least one of the gears is rotatably received, and wherein a magnet (M) for driving the gear or gears is located in the housing, wherein a second flushing passage (4) that extends up to the magnet (M) is located on the pressure side of the gears, **characterized in that** in the use state, in which the gear pump is vertical and the inflow (1) and the outflow (2) extend horizontally, the pump chamber (3) is located at the level of the inflow (1) and the outflow (2) is located below this level.

3. Gear pump in accordance with claim 1, **characterized in that** the gear pump is designed with the features of claim 2.

4. Gear pump in accordance with one of the preceding claims, **characterized in that** the inflow (1) is arranged higher than the outflow (2); and **in that** a downcomer (5) is provided that extends over the level difference or over a part thereof between the inflow (1) and the outflow (2), wherein the downcomer (5) is in fluid communication with the flushing passage or passages (4, 6).

5. Gear pump in accordance with one of the preceding claims, **characterized in that** the bearing position (7) is a sliding bearing.

6. Gear pump in accordance with one of the preceding claims, **characterized in that** the housing is made up of a plurality of individual layers.

7. Gear pump in accordance with claim 2 and claim 6 and with one of the claims 3-5, **characterized in that** one layer forms the base housing body and one layer forms a cover layer, wherein the inflow (1), the pump chamber (3) and the outflow (2) are arranged in the base housing body, and wherein the magnet (M) for driving the gear or gears is arranged in the cover layer.

8. Gear pump in accordance with claim 3 and one of the claims 4 to 7, **characterized in that** the first (6) and second (4) flushing passages are in fluid communication with one another.

9. Use of a gear pump in accordance with one of the claims 1 to 8 for conveying biological or medical fluids, in particular for conveying liquid to prepare a dialysis solution or for conveying a dialysis solution.

10. Use of a gear pump in accordance with one of the claims 1 to 8 in a blood treatment device, in particular in a dialysis machine.

11. Use in accordance with claim 9 or 10, **characterized in that** the gear pump serves as a degassing pump.

12. Blood treatment device, in particular a dialysis machine, having a gear pump in accordance with one of the claims 1 to 8.

## Revendications

1. Pompe à engrenage comportant un carter qui présente une entrée (1) pour le liquide à transporter et une sortie (2) pour le liquide transporté ainsi qu'un espace de pompe (3), dans lequel se trouvent la ou les roues dentées destinées au transport du liquide, au moins un point d'appui (7), dans lequel au moins une roue dentée est reçue de manière rotative, se trouvant dans le carter, l'entrée (1) se trouvant dans un autre plan que la sortie (2) et en ce qu'au moins un premier canal de balayage (6) est directement ou indirectement en liaison fluidique avec l'entrée (1) et/ou avec la sortie (2), ledit premier canal de balayage s'étendant jusqu'au ou jusqu'aux points d'appui (7), **caractérisée en ce que**, dans l'état d'utilisation, dans lequel la pompe à engrenage est en position verticale et l'entrée (1) et la sortie (2) sont horizontales, l'espace de pompe (3) se trouve à la hauteur de l'entrée (1) et la sortie (2) en dessous de ce niveau.

2. Pompe à engrenage comportant un carter qui présente une entrée (1) pour le liquide à transporter et une sortie (2) pour le liquide transporté ainsi qu'un espace de pompe (3), dans lequel se trouvent la ou les roues dentées destinées au transport du liquide, au moins un point d'appui (7), dans lequel au moins une des roues dentées est reçue de manière rotative, se trouvant dans le carter, et un aimant (M) destiné à l'entraînement de la ou des roues dentées se trouvant dans le carter, un second canal de balayage (4) se trouvant sur le côté refoulement des roues dentées, ledit second canal de balayage s'étendant jusqu'à l'aimant (M), **caractérisée en ce que**, dans l'état d'utilisation, dans lequel la pompe à engrenage est en position verticale et l'entrée (1) et la sortie (2) sont horizontales, l'espace de pompe (3) se trouve à la hauteur de l'entrée (1) et la sortie (2) en dessous de ce niveau.

3. Pompe à engrenage selon la revendication 1, **caractérisée en ce que** la pompe à engrenage est exécutée avec les caractéristiques de la revendication 2.

4. Pompe à engrenage selon l'une des revendications précédentes, **caractérisée en ce que** l'entrée (1) est disposée plus haut que la sortie (2) et **en ce qu'**il est prévu un canal de descente (5) qui s'étend sur la différence de niveau ou une partie de celle-ci entre l'entrée (1) et la sortie (2), le canal de descente (5) étant en liaison fluidique avec le ou les canaux de balayage (4, 6).

5. Pompe à engrenage selon l'une des revendications précédentes, **caractérisée en ce que** le point d'appui (7) est un palier lisse.

6. Pompe à engrenage selon l'une des revendications précédentes, **caractérisée en ce que** le carter est constitué de plusieurs couches individuelles.

7. Pompe à engrenage selon la revendication 2 et la revendication 6, et l'une des revendications 3 à 5, **caractérisée en ce qu'**une couche forme le corps de base de carter et une couche forme une couche de couvercle, l'entrée (1), l'espace de pompe (3) et la sortie (2) étant disposés dans le corps de base de carter et l'aimant (M) destiné à l'entraînement de la ou des roues dentées étant disposé dans la couche de couvercle.

8. Pompe à engrenage selon la revendication 3 et l'une des revendications 4 à 7, **caractérisée en ce que** le premier (6) et le second (4) canal de balayage sont en liaison fluidique l'un avec l'autre.

9. Utilisation d'une pompe à engrenage selon l'une des revendications 1 à 8 pour le transport de liquides biologiques ou médicaux, en particulier pour le transport de liquide destiné à la fabrication d'une solution de dialyse ou pour le transport d'une solution de dialyse.

10. Utilisation d'une pompe à engrenage selon l'une des revendications 1 à 8 dans un appareil de traitement du sang, en particulier un appareil de dialyse.

11. Utilisation selon la revendication 9 ou 10, **caractérisée en ce que** la pompe à engrenage sert de pompe de dégazage.

12. Appareil de traitement du sang, en particulier appareil de dialyse comportant une pompe à engrenage selon l'une des revendications 1 à 8.
